# EUROPEAN PATENT APPLICATION

(11) **EP 1 589 449 A1**
(43) Date of publication of application: **26.10.2005**
(21) Application number: 04009638.0
(22) Date of filing: 23.04.2004
(51) Int. Cl.: G06F 17/60, G06F 17/30, G06F 19/00

(54) **Website messaging system for public health information**

(71) Applicant: Joseph Sameh, Wilmette, IL 60091 (US)
(72) Inventor: Joseph Sameh, Wilmette, IL 60091 (US)
(74) Representative: Schmid, Wolfgang

(57) **Abstract**

A method and apparatus are provided for providing information to a public health agency from a website of a physician. The method includes the steps of detecting entry of a symptom from a patient through the website, comparing the detected symptom with a public health care criteria and forwarding the detected symptom to the public health agency when the detected symptom meets the public health care criteria.

## Description

### Field of the Invention

The field of the invention relates to physician/patient contact and more particularly to methods of forwarding messages from a patient to a physician.

### Background of the Invention

This patent application is a continuation-in-part of U.S. Patent Application Number 10/115,393, filed on April 3, 2002 (pending).

Good communication between doctor and patient has always been an important part of healthcare. While face-to-face communication has always been the best form of communication, it is also often necessary for physicians to remain available after hours for emergencies and other patient concerns.

The traditional method of contacting a physician after hours has been through an answering service. Answering services answer calls directed to the physician and take messages. The physician may periodically call the answering service to pick up his messages. Alternatively, the answering service may page the physician for each message, if the physician has a pager.

Alternatively, a need for good communication also exists between the medical community and public health agencies (e.g., the Center for Disease Control (CDC) in Atlanta, Georgia). Where the public health is concerned, the CDC typically relies upon emergency room reporting as a means of detecting public health problems.

While the use of emergency room records has been effective, only the most extreme health care problems are often seen in emergency rooms. Other less severe problems may not be reported because the patient may wait to see his/own physician. Where the patient is required to wait for an appointment, the problems may ameliorate with time or may be subsumed by other health care problems.

However, less severe health care problems often evolve into other more complicated problems and health care issues that are difficult to trace back to a common source. Further, less severe health care issues are being increasingly related to long-term quality of life issues.

In addition, the specter of bioterrorism raises further issues about the wisdom of ignoring less severe health care issues. Because of the impact of less severe health care issues upon the long-term health of the public in general, a need exists for a more effective method of reporting such problems to public health agencies.

### Summary

A method and apparatus are provided for providing information to a public health agency from a website of a physician. The method includes the steps of detecting entry of a symptom from a patient through the website, comparing the detected symptom with a public health care criteria and forwarding the detected symptom to the public health agency when the detected symptom meets the public health care criteria.

### Brief Description of the Drawings

FIG. 1 is a block diagram of a website messaging system under an illustrated embodiment of the invention;

FIG. 2 is a physician selection screen that may be used by the system of FIG. 1;

FIG. 3 is a classification screen that may be used by the system of FIG. 1;

FIG. 4 is an existing patient screen that may be used by the system of FIG. 1;

FIG. 5 is a new patient screen that may be used by the system of FIG. 1;

FIG. 6 is a pharmacist screen that may be used by the system of FIG. 1;

FIG. 7 is a medical staff screen that may be used by the system of FIG. 1;

FIG. 8 is a priority selection screen that may be used by the system of FIG. 1;

FIG. 9 is a key word entry screen that may be used by the system of FIG. 1; and

FIG. 10 is a routing priority screen that may be used by the system of FIG. 1.

### Detailed Description of an Illustrated Embodiment

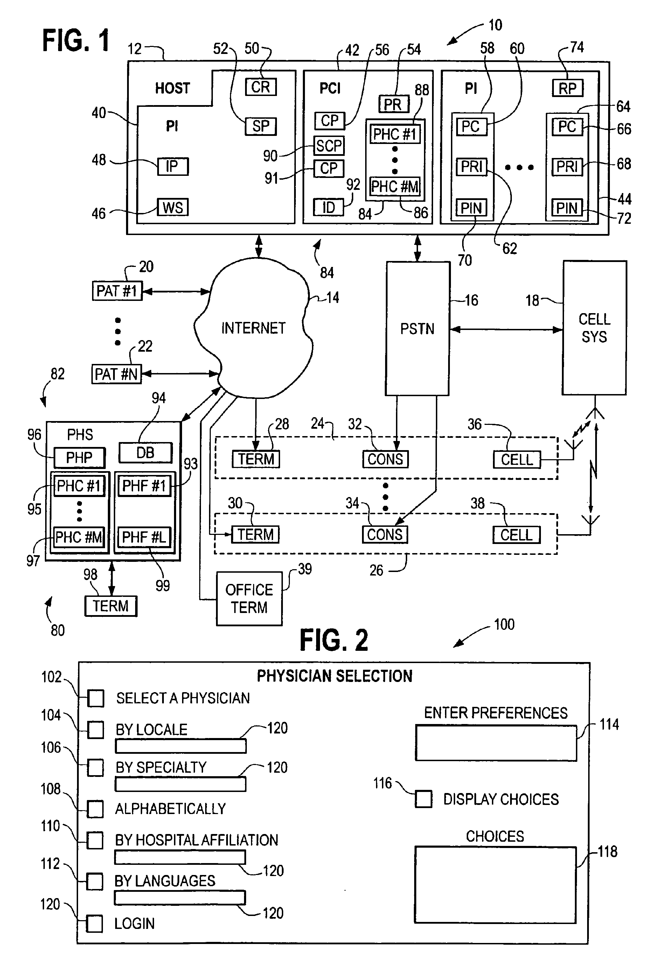

FIG. 1 is a block diagram of a website messaging system 10 shown generally in accordance with an illustrated embodiment of the invention. The website messaging system 10 may be used to forward messages from patients to their attending physicians under any of a number of predetermined message formats and patient conditions specified by the attending physician.

The system 10 may also be used by associates of the physician (e.g., labs, pharmacies, etc.) to forward messages about patients to the attending physician.
Because of the flexibility of the system 10 in handling messages the term "patient" or "requestor" is often used herein generically to refer to any of patients, associates of the physician or to other physicians passing messages through the system 10.

The system 10 may include a number of different interfaces 40, 42, 44. A first interface 40 may function as a patient interface that is able to provide information to and receive information from a patient 20, 22. One of the primary functions of the patient interface 40 is to identify a patient's attending physician and to function as an interface between the patient and the identified attending physician from among the many other physicians that may also use the system 10.

A second interface 42 may function as a processing center interface. The processing center interface 42 may function to receive information from the patient 20, 22 and determine a priority of the message.

A third interface 44 may function as the physician interface 44. The physician interface 44 functions to deliver the messages based upon the determined nature of the request and a set of delivery instructions provided by the physician.

The physician interface 44 may include a number of physician's records 58, 64, including one record for each physician using the system 10. Each record 58, 64 may include a physician's priority criteria 62, 68 and also a set of physician's routing instructions 62, 68 for routing requests to the physician.

The physician interface 44 may function to deliver messages to physicians under any of a number of communication formats (e.g., Internet, voice channel through the public switched telephone network (PSTN), voice channel through a cellular system, data through a cellular system, pager, palm pilot etc.). Because of the multiplicity of communication channels through which a request may be delivered, a communication sphere 24, 26 may be defined for each physician.

The communication sphere 24, 26 may be defined in a metaphysical sense as the physical space proximate the physician that may contain one or more communication devices. For example, if a first physician and his associated communication sphere 24 (the physician and communication sphere sometimes referred to hereinafter, together, as the "physician 24") were located in his office (either in his home or in his clinic), then he may have access to a computer terminal 28, a telephone console 32 and a cell phone 36, all at the same time. In this case, the communication sphere 24 of the first physician may include the computer terminal 28, the telephone console 32 and the cell phone 36, as shown in FIG. 1.

If the physician 24 should leave his office and take his cell phone 36 with him, then the physician's communication sphere 24 would only include the cell phone 36. Further, if the first physician 24 should leave his office and enter the office of a second physician 26, then the communication sphere 24 of the first physician may also include the communication devices 30, 34, 38 that are also within the communication sphere 26 of the second physician.

In order to accommodate the mobile nature of physicians, the physician interface 44 may be easily altered to include the changing range of communication devices located within the communication sphere 24, 26 of the physician. It should also be noted that the physician interface 44 may be altered to include the communication devices subscribed to by the physician or the communication devices of other physicians or non-physicians. In addition, while only two communication sphere 24, 26 are shown in FIG. 1, any number of spheres 24, 26 and any number of communication devices 28, 30, 32, 34, 36, 38 may be accommodated by the system 10.

Turning first to the patient interface 40, an explanation will first be offered of the interface 40 and how it functions to collect relevant information.
Following a description of the patient interface 40, an explanation will be provided of the processing center interface 42 and of the physician interface 44.

In order to facilitate the simple and convenient use of the patient interface 40, the interface 40 may allow a patient 20, 22 to contact his physician through a web site (e.g., with a URL of "NeedMyDoctor.com"). Contacts through the web site 46 may be routine (e.g., making an appointment, obtaining a renewal of a prescription, etc.) or on a more urgent basis (e.g., an emergency).

Identification of a patient's physician may be accomplished automatically in the case of an existing patient by storing an identifier of the attending physician as a cookie in a browser of a computer terminal 20, 22 of the patient. In the alternative, new patients may be offered the opportunity to select a physician based upon the needs and preferences of the patient 20, 22.

Upon accessing the web site 46, an identity processor 48 may attempt to identify the patient 20, 22. Identification of the patient 20, 22 may be accomplished by retrieving a URL of the party accessing the web site 46 or by retrieval of any cookies present within the browser of the accessing party 20, 22. In either case, the URL or cookies may be compared with the contents of a set of customer records 50 to identity any physicians that the patient 20, 22 may have previously selected.

In either case, a screen (web page) 100 (FIG. 2) may be downloaded to a browser of the patient or other requestor 20, 22. If the patient 20, 22 has previously used the system 10 and has previously selected a physician, then any selected physicians may be displayed within a CHOICES box 118.

If the patient 20, 22 has previously used only a single physician, then a name, image and biography of the physician may be displayed in the choice box 118. The patient 20, 22 may select this physician by activating a SELECT A PHYSICIAN button (softkey) 102 or by activation of an ENTER button on his terminal 20, 22.

If the patient 20, 22 has previously used or selected more than one physician, then a list of the names of previously selected physicians may be displayed in the CHOICES box 118. The patient 20, 22 may either select a physician from among those displayed in the CHOICES box 118 or select yet another physician by activation of one or more criteria selection boxes 104, 106, 108, 110, 112. If the patient 20, 22 selects from among the list of previously selected physicians within the box 118, then a name and image of the physician may be displayed and the patient 20, 22 may be proceed as described above.

If the patient 20, 22 chooses to select another physician, then he may select the other physician based upon any of a number of different criteria (e.g., locale, medical specialty, hospital affiliation, language ability, etc.). Further, the patient 20, 22 may refine his search by using a preferences window 114.

For example, the patient 20, 22 may enter an "A" in the preferences window 114 and activate a LOCALE button 120. Alternatively, the patient 20, 22 may activate the LOCALE button 104, enter an "A" in the box 114 and select the DISPLAY CHOICES button 116. In response, a selection processor 52 within the patient interface 40 may provide the patient with a set of choices on locale that begin with "A" (e.g.: Alabama; Alaska; Albany, New York, etc.).

The patient 20, 22 may make a selection and proceed to another search criteria. Upon making a selection, the selection may appear in a criteria display 120 associated with the criteria.

If the patient 20, 22 should then activate the BY HOSPITAL AFFILIATION button 110, then a list of hospitals in the selected locale may be displayed in the CHOICES box 118. As above, the patient 20, 22 may make a selection and the selection may appear in the box 120 associated with the selected criteria.

The patient 20, 22 may then select a specialty and, possibly a language preference. The patient 20, 22 may then select an ALPHABETICALLY button 108 to view a list of physicians under the combination of criteria chosen.
The patient 20, 22 may select a physician from the list shown in the CHOICES area 118 and activate the SELECT A PHYSICIAN button 102 to complete the process.

As a much simpler alternative, the patient may simply enter a physician's name in the ENTER PREFERENCES box 113 and activate the SELECT A PHYSICIAN box 102. The name and image of the physician may appear in the CHOICES box 118. The patient 20, 22 may then review his choice and then activate the SELECT A PHYSICIAN box 102 a second time to complete selection of the physician.

The ability to enter a physician's name is a tremendous advantage for people who are traveling and experience a medical problem. In this case, the requestor 20, 22 may simply go to any computer and access the website 46 remotely.

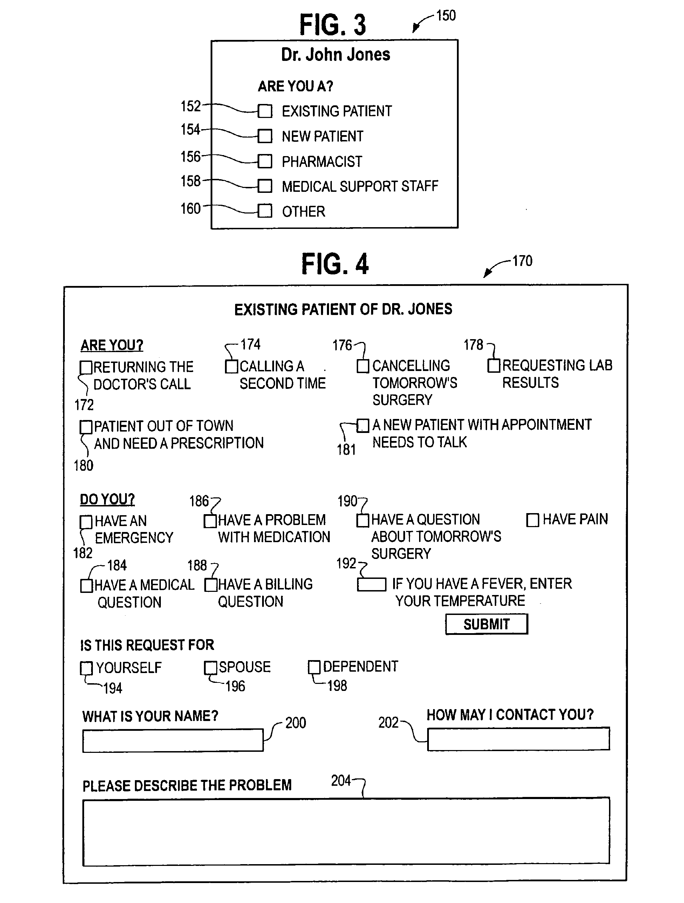

Following selection of a physician, the web page 150 (FIG. 3) may be downloaded to the patient 20, 22. Included within the web page 150 may be a number of classification buttons 152, 154, 156, 158, 160 for each message. While any method of classification may be used one method divides the messages according to whether the source is an existing patient, a new patient, medical support staff or other. Activation of an EXISTING PATIENT button 152 may be used to indicate that the patient 20, 22 is already under the care of the physician. Activation of an NEW PATIENT button 154 may be used to indicate that the patient 20, 22 has never seen the physician. Activation of the PHARMACIST button 156 may be used to indicate that the requester 20, 22 may be a pharmacist with a question about a prescription. Activation of the MEDICAL SUPPORT STAFF button 158 may indicate a message from another physician or a message from a person providing support services to the physician. Finally, activation of the OTHER button 158 may be used for any other purpose selected by the physician.

If the patient 20, 22 should activate the EXISTING PATIENT button 152, then the screen 170 of FIG. 4 may be downloaded to the patient 20, 22. Within the screen 170, a first set of boxes (softkeys) 172, 174, 176, 178, 180, 181 may be provided for entering an overall reason for the message. A second set of boxes 182, 184, 186, 188, 190, 192 may be provided for determining a physical state of the patient. A text entry window 192 may be provided for entry of a patient temperature.

Another text box 200 may be provided for entry of a name of the requestor 20, 22. A text box 202 may also be provided for entry of a communication path (e.g., telephone number, e-mail address, pager number, etc.) through which the physician can reach the requestor 20, 22. The requestor 20, 22 may also be able to identify through a set of softkeys 194, 196, 198 whether the requestor is the patient or whether the patient is a relative. A text box 182 may be provided for entry of a description of the problem.

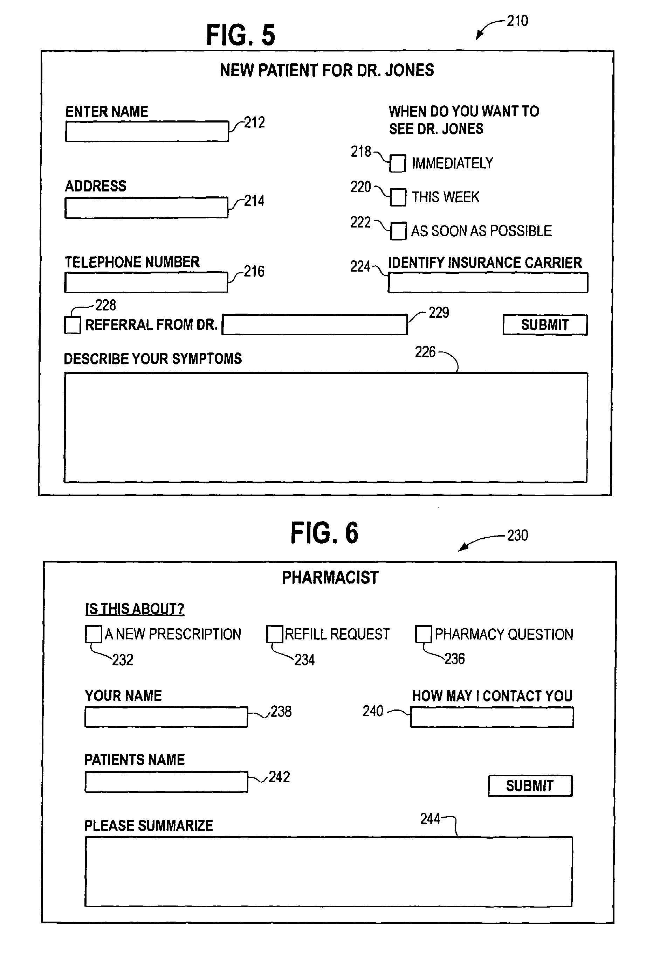

If the patient 20, 22 should activate the NEW PATIENT softkey 154, then a screen 210 of FIG. 5 may be downloaded to the requestor 20, 22. Text boxes 212, 214, 216 may be provided for entry of a name, address and contact information of the patient. A softkey 228 and textbox 229 combination may be used to identify the patient as a referral and the source of the referral. Selection buttons 218, 220, 222 may be provided for scheduling an appointment. A text box 224 may be provided for entry of insurance information. Finally, a large text box 226 may be provided for entry of descriptive information regarding the reason for the appointment.

If the requestor 20, 22 should activate the PHARMACIST softkey 156 on FIG. 3, then the screen 230 of FIG. 6 may be downloaded to the requestor 20, 22. Within the screen 230, the requestor 20, 22 may be provided with text boxes 238, 240 for identification of the requestor and to enter contact information. A set of softkey boxes 232, 234, 236 may be provided for the requestor to differentiate between questions about new prescriptions, refills and general pharmacy questions.

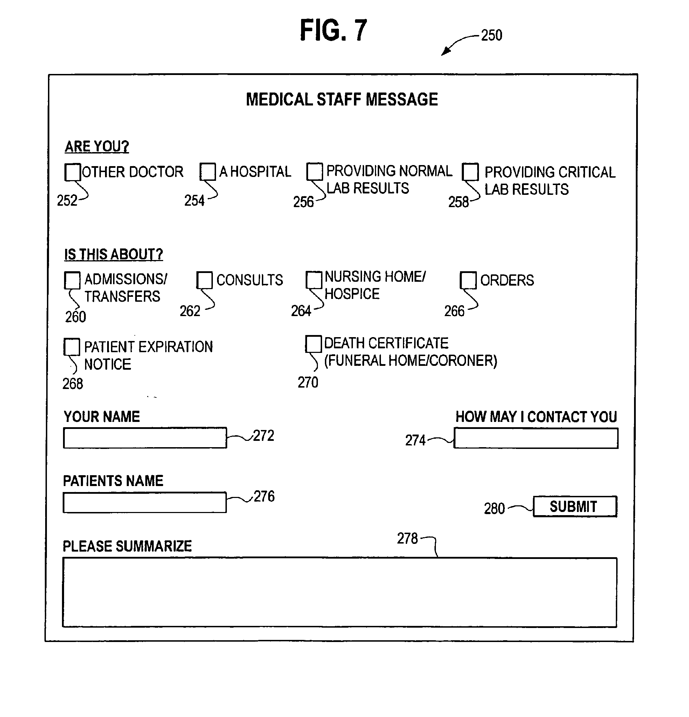

If the requestor 20, 22 should activate the MEDICAL SUPPORT STAFF button 158, then the screen 250 of FIG. 7 may be downloaded to the requestor 20, 22. Within the screen 250, the requestor 20, 22 may be asked to enter his name and contact information in one set of boxes 272, 274. Another set of boxes 252, 254, 256, 258, 260, 262, 264, 266, 268, 279 may be provided for entry of a context of the request.

In addition, a text box 276 may be provided for entry of a patient's name. Another text box 278 may be provided for entry of a message regarding the patient. A SUBMIT button 280 allows the requestor 20, 22 to return the message to the system 10.

If the requestor 20, 22 should activate the OTHER box 160, then a single blank text box may be downloaded. The OTHER button 160 may be used for any of a number of purposes as described in more detail below.

Once the requestor 20, 22 has selected a physician and made whatever entries are appropriate through the text boxes of FIGs. 3-7, the requestor 20, 22 may activate a SUBMIT button. Activation of the SUBMIT button causes the terminal of the requestor 20, 22 to compose a requestor (patient) message with the entered information disposed within predetermined data fields of the message. The composed message may be transferred to the processing center interface 42. Within the processing center interface 42, a content processor 56 may detect and process each message to determine a nature and content of the request based upon the information elements provided through the web pages (e.g., the identity of the requestor, any classification information provided through the classification buttons and any text information received through the text boxes).

Based upon the determined nature and content of the request, a relative importance may be assigned to the request, based upon a subjective criteria provided by the physician. The criteria is necessarily subjective because the relative importance of information elements varies from one physician's practice to another physician's practice and in accordance with the preferences of one physician over another physician.

For example, physicians with a practice limited to surgery may only classify messages from hospitals or other surgeons or patients with post operative problems as significant enough to justify an expedited message to the physician. Other messages to the surgeon may be regarded as much less important.

In the alternative, a physician with a practice limited to pediatric care may only consider children with high fevers, broken bones or severe bleeding as important. Other requests related to less severe trauma may be considered to be less important. Further the criteria for routing messages to a physician may be changed based upon the time of day.

For example, during normal office hours, all messages may be routed to a nurse or other assistant at a clinic or hospital where the physician is normally to be found during those hours. After normal office hours, messages may be routed to the physician only when the physician is on call. When the physician is not on call, any requests to the physician may be automatically routed to another designated physician.

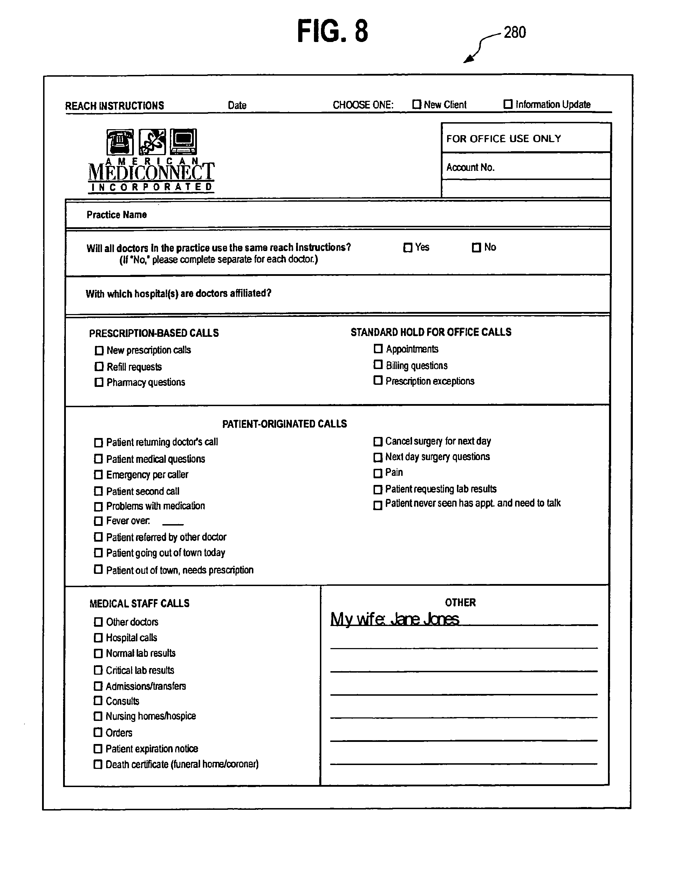

Based upon the nature of the request, the content processor 56 may route the message, by comparing an information content with a set of threshold values provided by the physician. FIG. 8 provides a screen 280 that may be downloaded to a terminal 28, 30 of a physician 24, 26 for purposes of setting threshold values for forwarding messages. The screen 280 may be downloaded to a physician during initial registration with the system 10 or at any time thereafter to change the routing format.

The screen 280 of FIG. 8 may be used by the physician to establish a multi-level message forwarding methodology. Under one embodiment, the box shown along the left side of each subject matter listing in screen 280 may be a text box where a number value indicating priority may be explicitly entered. For purposes of simplicity, the system 10 will be described as being based upon a two-level system of priorities. However, any number of priority levels could be used.

Also, for purposes of simplicity, the use of the screen 280 will be described using a system of default levels. Instead of entering a number in the box, the boxes may be used as softkeys. If the softkey is activated by the physician, then the subject matter of that softkey will be given the highest priority. If the softkey is not activated , then the subject matter will be given the second, lower level of priority.

At a highest, first level of importance, messages may be routed directly to a physician's sphere 24, 26. At the second level, requests may be routed to a secondary destination (e.g., an office of the physician).

For example, checking the box in the upper left corner (labeled "New prescription calls") would result in all messages from pharmacists about new prescriptions being given the highest priority and routed directly to the physician. Further, checking the box in the bottom of the right-side (labeled "Patient never seen has appt. and need to talk") would result in messages from first time patients begin routed on the first level.

In general, the solicitation and processing of messages from requestors 20, 22 within the content processor 56 may be controlled by a physician's criteria obtained by the system 10 through screen 280 and stored in a file 60, 66 for each physician. Once a physician 24, 26 has identified a routing criteria (e.g., using screen 280), the content processor 56 may retrieve the routing criteria for that physician 24, 26 and use the criteria for message routing.

As each webpage 150, 170, 200 is completed and returned to the system 10, the content processor 56 may examine the content of the webpage 150, 170, 200 under the criteria provided by the associated physician 24, 26. A determination of the nature and importance of the message may occur on any of a number of different levels.

On a first level, the processor 56 may determine the type of message based upon the information elements provided through screens 170, 210, 230, 250. Following a determination of the type of message, the content processor 56 may perform an element-by-element comparison between selected items for that message type on screen 280 and the content of the message.

The message type of STANDARD HOLD FOR OFFICE CALLS would always be classified as a low priority unless the physician indicated otherwise. If the physician has selected "Appointments", then messages from new patients where the softkey 154 is activated would be given a high priority. The selections for "Billing questions" and "Prescription exceptions" may be given similar treatments.

Messages from patients may be processed somewhat differently. For example, a physician may not only select the option "Fever over ", but may also specify a priority limit for the fever. Detection may be accomplished by a comparison of the numeric value entered through the text box 192 and the threshold value provided by the physician. Alternatively, the physician may specify any fever within one week post operatively.

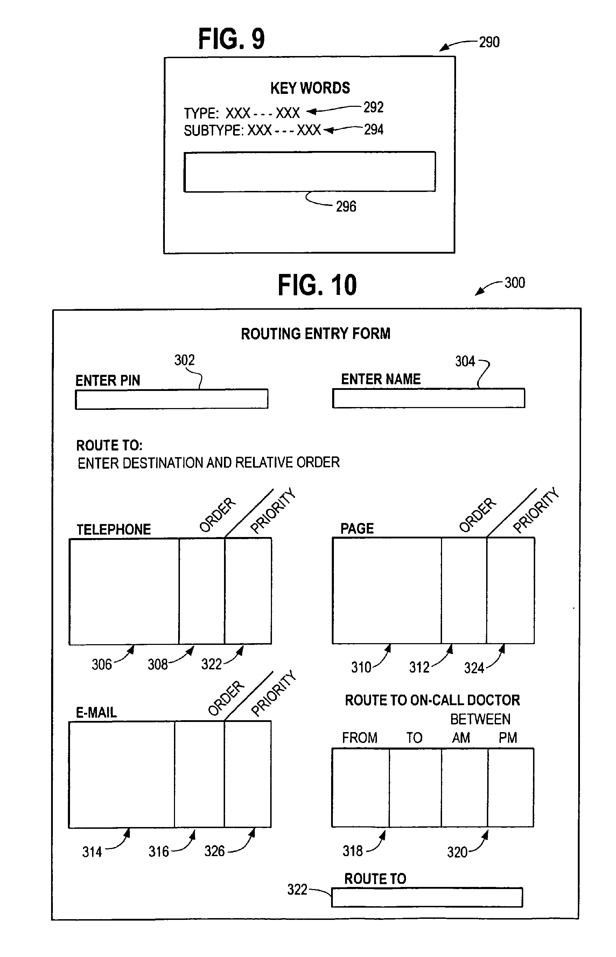

On another level, the physician 24, 24 may also set a criteria for message routing based upon key word searching using words entered through any of the text boxes 202, 204, 226, 238, 242, 244, 272, 272, 276, 278. For example, the physician 24, 26 may go to screen 280 of FIG. 8 and double click on any element or sub-element to bring up a text box associated with that element. For example, the physician 24, 26 may double click on the "OTHER" category on screen 280. In response, the text box 290 of FIG. 9 may be downloaded to the physician's terminal 28, 30. Within the text box 290, a first line 292 may indicate the type of text box as being "OTHER". Since the "OTHER" category does not have a sub-element, the second line 294 may be blank.

Upon entering the text box 290, the physician 24, 26 may enter his wife's name (e.g., "Jane Jones") or some other word identifying his wife. Entry of his wife's name as a criteria for the "OTHER" category allows any message sent under the "OTHER" classification and that includes his wife's name or identifier to be given a high routing priority.

To use the facility, the physician's wife would enter the web site 46, type her husband's name in text box 114 and activate "SELECT A PHYSICIAN". On the next downloaded screen 150, the wife would select "OTHER". In response, a blind screen would appear within which the physician's wife may type "From: Jane Jones" and a message. Upon receiving the message, the content processor 56 would compare the key words "Jane Jones" with the content of the message and recognize the matching words "Jane Jones" present in the message header. Based upon the match, the content processor 56 would route the message at the highest priority level.

Alternatively, the physician may also click on "PATIENT-ORIGINATED CALLS" on screen 280 and enter a patient's name. Based on key word searching, any message from that patient would be routed at the highest priority.

The use of key word searching allows a physician to customize call routing for any time period (e.g., 2 hours, 2 days, permanently, etc.) to meet the needs of critically ill patients. Alternatively, if the physician is part of a surgical team waiting for a transplant donor, key word searching could be used to automatically identify messages from other members of the surgical team.

Once the content processor 56 determines an information content and priority level of a message, the content processor 56 may transfer the message to a routing processor 74 within the physician's interface 44. Within the physician's interface 44, the routing processor 74, may retrieve a set of routing instructions 68 based upon the priority level determined by the content processor 56.

Located within the routing instructions 68 may be a prioritized list of communication devices within the physician's communication sphere 24, 26. Messages may be routed to the physician 24, 26 based upon the entries within the prioritized list.

FIG. 10 depicts a routing webpage 300 that may be downloaded from the web site 46 to a terminal 28, 30 of the physician. The routing webpage 300 may be accessed by first accessing the physician identification webpage 100 (FIG. 2) and activating LOGIN softkey 120.

Within the webpage 300, the physician may first be required to enter his personal identifier number (PIN) into a first box 302. Upon entering his PIN number the terminal 28, 30 may upload the PIN to the routing processor 72 where the PIN is compared with the PINs 70, 72 of other physicians using the system 10.

If a match is found, the routing processor 74 may download a name of the physician to be displayed in a first box 304 and a current content of the physician routing instructions 62, 68 to be displayed in other boxes 306, 310, 314.

Included with each routing destination is an ordering number 308, 312, 316 and priority ranking 322, 324, 326. The ordering number indicates the relative position of the routing destination in the routing list for any particular priority level. For example, at the highest priority level, if the physician wishes to be paged first, then the pager number would appear at a top of a list in box 310 with a number "1" shown opposite the pager number in an order list 312 and a "1" in the priority ranking. If the physician wishes to simultaneously receive a hardcopy of the message on his computer 28, 30, then an e-mail address of his computer may appear on the top of list in an e-mail box 314 opposite another number "1" in the order box 316 and a "1" in the priority ranking. Once the physician downloads the screen 300, he may make new entries, delete old entries or change the order at will.

In addition to setting up a routing list, the physician 24, 26 may also set up a schedule when he/she is not to receive messages (i.e., the physician is not on call). As shown, the physician simply enters his dates and hours when the physician is not on call and when calls should be routed to another physician. The entry of time periods into boxes 318, 320 simply causes messages to be routed to an alternate physician in an on call list maintained within the system 10. An identifier of the alternate physician may be entered into a "ROUTE TO" text box 322.

Delivery of the messages may occur under any of a number of different formats. For example, if the physician's computer 28, 30 is the destination of a message, then the delivered message may have the same format as shown in FIGs. 2-7. Alternatively, the format of FIGs. 2-7 may be changed to delete unnecessary information.

If the destination is a cell phone or a telephone, then a voice synthesizer may be used to present the messages of FIGs. 2-7 under a predefined audio format. Alteration of the call list based upon screen 300 may also be accomplished using a telephone, the voice synthesizer and keypad selection on the telephone.

Once a message has been delivered to the physician 24, 26, the routing processor 74 may send a message back to the patient interface 40 and patient 20, 22 confirming receipt of a high priority message by the physician. Where the physician responds to the patient's message through the system 10, the routing processor 74 may also calculate an average time for the physician to respond. In such cases, the routing processor 74 may also include an estimate of the expected time for the physician to respond in the message to the patient 20, 22.

In another embodiment of the invention, the website messaging system 10 is provided with a public health data reporting interface 80 that automatically transfers information to a public health information recipient. As used herein, a public health information recipient may be a governmental public health agency, a pharmaceutical company, a developer of medical devices or other reporting or monitoring agency. Under a preferred illustrated embodiment, a public health information recipient is a governmental public health agency, a pharmaceutical company or a developer of medical devices.

Under one illustrated embodiment, the public health information recipient is a governmental public health agency (e.g., a local public health agency, the CDC in Atlanta, GA, etc.) 82. A data collection portion 84 of the reporting interface 80 functions to compare symptoms of patients with one or more public health criteria and automatically report suspicious symptoms to the public health agency 82 where a patient's symptoms meets a reporting criteria.

The reporting interface 80 may have great value in helping to identify public health problems on any of a number of different levels. For example, at its most basic level, the reporting interface 80 may be able to identity influenza outbreaks at a patient level rather than at the treatment level. Further by aggregating reports among many physicians, the public health agency 82 may use the information provided through the reporting interface 80 to identify outbreaks that would not be recognizable to individual physicians. For example, in the case of a disease with diffuse symptoms, the diffuse symptoms may mask the common nature of the disease to individual physicians because of the physician's relatively small number of patients. However, by being able to receive and examine reports from large numbers of cases, the public health agency 82 may be able to correlate and identify the common nature of the disease.

Further, by being able to collect information automatically at the source, the public health agency 82 may be able to react in a manner never before possible. For example, in the case of an attack by a bioterrorist, the public health agency 82 may be able to pinpoint the epicenter of the attack even before the nature of the attack has been identified.

Further, by being able to collect information at the patient level, a public health agency 82 may be able to more effectively monitor the dynamic nature of public health. In being able to more effectively monitor the dynamic nature of public health, the public health agency 82 may be able to more effectively utilize scarce resources in times of emergency.

Further, any manner of criteria may be used to identify suspicious symptoms. In general, the criteria may be based upon any of a number of descriptive terms (e.g., vomiting, rashes, pustules, inflammation, headaches or bodyaches, discolorations, bleeding, discharges, diarrhea, dilation of the eyes, lack of appetite, etc.) or measurable parameters (e.g., body temperature, heart rate, weight loss, etc.).

Turning now to the reporting interface 80, an explanation will be provided of the operation of the reporting interface 80. As may be noted from FIG. 1, the reporting interface 80 has a first and second portion. The first portion (referred to as the data collection portion) 84 may reside within the patient interface 42 of the website messaging system 10. The second portion (referred to as the control portion of the reporting interface) 82 may reside with the public health agency.

The data collection portion 84 may consist of a symptom comparison processor 90 that operates under the control of a reporting criteria of the public health agency 82. The reporting criteria may consist of one or more files 86, 88 located within a memory 84 of the processing interface 42. The comparison processor 90 functions to compare the contents of the information entered through the windows 170, 210 with the criteria 86, 88 and when the contents of a window 170, 210 meets the criteria, to report the contents of the window to the public health agency 82.

Under one simple example, the public health agency 82 may provide a criteria (e.g., file 86) for identifying influenza. The criteria 86 for identifying influenza may be an elevated temperatures and diarrhea. In this case, criteria 86 may specify a temperature above some predetermined threshold value in conjunction with the presence of the word "diarrhea", "runs" or some other colloquialism having the same meaning.

For this example, as the patient message generated in response to each window 170, 210 is received, the processor 90 may process the information contained within the predetermined information fields of the patient message to identify patients that meet the criteria. In the case of the window 170, the processor 90 may retrieve each temperature entry provided through window 192 and compare the entry with the threshold value in the file 86. If the entry equals or exceeds the threshold value, then the processor 90 may proceed to do a word search of the window 204 for the presence of any word that has the same meaning as the word "diarrhea".

In the case of the window 210, the process may proceed in a similar manner except the word search may also include a search for a three digit number within a certain distance (e.g., three words) of the word "temperature". Where a temperature is found, the temperature may be compared with the threshold value. If the value exceeds the threshold, then an additional search may be performed for indications of diarrahea. If both factors are found, then the symptoms may be determined to have met the criteria 86.

While the criteria for influenza represents a very simple example, other more complex examples may also be offered. Further, the criteria may not require the presence of every descriptive element of the criteria to cause transfer of the information to the public health agency 82. For example, some patients afflicted with a particular disease may not exhibit all possible symptoms. As a result, the criteria 86, 88 used in any particular situation may specify a minimum number of required and alternate elements for meeting any particular criteria 86, 88.

In each case where the criteria is met (e.g., the temperature exceeds the threshold and diarrhea is present in the case of influenza), the comparison processor 90 may transfer the information entered by the patient to a communication processor 91. The communications processor 91 may compose a message for transfer through the Internet 14 to the public health agency 82. The message may include the information entered by the patient as well as additional information. The additional information may include an identifier of the criteria 86 met as well as a location identifier 92 of the source of the message. An identifier of the physician and patient may also be included within the message, although the identifier of the patient may be suppressed or encoded for privacy reasons.

Upon receipt of the message by the public health agency 82, the message may be entered into a database 94. Once entered into the database, a processor of the agency 82 may process the information using conventional methods.

The criteria 86, 88 used for evaluating symptoms may be downloaded and used within the website 10 under control of the public health agency 82. One or more terminals 98 may be provided for creation and entry of screening profiles (criteria) 95, 97. Once created, the profiles 95, 97 may be downloaded to all website messaging systems 10 or only to specific systems 10 within a particular geographic, cultural or environmental area.

The ability to target and isolate particular areas for particular criteria allows any of a number of public health issues to be addressed on a dynamic basis. For example, in the event of a volcanic eruption (e.g., Mt. St. Helens), the websites 10 of downwind physicians may be provided with a criteria directed to identifying and gathering data associated with respiratory problems associated to volcanic dust. Similarly, in the case were an inversion layer prevents dispersion of air pollution over San Diego, CA, a similar criteria for respiratory problems could be downloaded to specific targeted areas.

If an anthrax threat were to be directed to a particular city or the country as a whole, then a criteria may be developed and downloaded in response. In the case of anthrax, the criteria may be skin lesions alone or skin lesions associated with respiratory distress.

In another embodiment, the reporting system 80 could be used as a tool to isolate contagious diseases without causing panic among the public. For example, if a single case of a particular disease (e.g., hemoragic fever) were diagnosed in a particular area, then the public health agency 82 may create a criteria (e.g., file 95) and download that criteria 95 to the websites 10 of physicians where the disease were discovered. Since the reporting interface 80 could collect a body of extremely relevant information without the express knowledge of the public, the public health agency 82 could evaluate and allocate resources without spreading panic.

In addition, since the website 10 would operate on a 24/7 basis, the data collected through the reporting interface 80 would be virtually real-time. Further, since a patient would be expected to call his doctor far sooner than he would go to an emergency room, it would be expected that information collected through the public health information interface 80 would be far more relevant to the progression of a epidemic (or pandemic) than any other prior art process.

In another embodiment of the invention, the public health reporting interface 80 may be used for adverse event reporting. In this case, the reporting interface 80 may be used to detect adverse events associated with clinical studies (e.g., drug testing, medical appliance testing, etc.).

In this case, the criteria 86, 88 created through the terminal 98 may include the name of a particular drug or appliance by itself or the criteria 86, 88 may also include known or possible symptoms that could be associated with the use of the drug or device. The criteria 86, 88 may be downloaded to all websites messaging systems 10 or only those that participate in clinical trials of the drug or device.

As each patient message is received by the data collecting portion 84, the message is compared with the criteria 86, 88. The comparison processor 90 may compare each message with the criteria related to the drug or device. Where a match is found with the criteria, the comparison processor 90 may transfer the message and criteria to the communication processor 91. The communication processor 91 may compose an adverse event reporting message to the public health agency 82 that includes the content of the patient message and the criteria met by the message.

Once received by the public health agency 82, the report message may be stored in a file 93, 99 associated with the drug or device. A terminal 98 of the agency 82 may be used to acquire information on adverse events across all clinical research sites or by clinical study protocol. Compilation and reporting of these events may be available on a real-time basis protocol wide to pharmaceutical companies or medical device developers by remotely accessing the agency database.

Further adverse event data may be accessed through the terminal 98 or a remote terminal at any time or forwarded immediately to an appropriate organization for action by an appropriate event processing module operating within the processor 96. In addition to aggregating event data for a single study, multiple studies working on the same compound can also be tracked and reported in real time.

A specific embodiment of a method and apparatus for collecting public health information or adverse event data has been described for the purpose of illustrating the manner in which the invention is made and used. It should be understood that the implementation of other variations and modifications of the invention and its various aspects will be apparent to one skilled in the art, and that the invention is not limited by the specific embodiments described. Therefore, it is contemplated to cover the present invention, any and all modifications, variations, or equivalents that fall within the true spirit and scope of the basic underlying principles disclosed and claimed herein.

## Claims

1. A method of providing information to a public health agency from a website of a physician, such method comprising the steps of:
detecting entry of a symptom from a patient through the website;
comparing the detected symptom with a public health care criteria; and
forwarding the detected symptom to the public health agency when the detected symptom meets the public health care criteria.

2. The method of providing information to the public health agency as in claim 1 further comprising downloading a webpage to the patient for entry of symptoms through the website.

3. The method of providing information to the public health agency as in claim 2 wherein the step of detecting entry of the symptom from the patient further comprises retrieving patient information from predetermined fields of a message from the patient.

4. The method of providing information to the public health agency as in claim 3 further comprising performing word searching on the information from the patient.

5. The method of providing information to the public health agency as in claim 4 further comprising using words from the healthcare criteria as search terms for the word searching.

6. The method of providing information to the public health agency as in claim 5 wherein the step of forwarding the detected symptom further comprises forwarding the information entered by the patient that met the public health care criteria.

7. The method of providing information to the public health agency as in claim 5 further comprising defining the public health care criteria as being a fever above a predetermined value.

8. The method of providing information to the public health agency as in claim 1 further comprising defining the public health care criteria as being a plurality of disease profiles.

9. The method of providing information to the public health agency as in claim 1 further comprising defining the public health care criteria as an adverse event related to the patient's use of a drug or medical device.

10. The method of providing information to the public health agency as in claim 9 further comprising reporting the adverse event to a pharmaceutical company sponsoring the drug or to a developer of the medical device.

11. The method of providing information to the public health agency as in claim 1 further comprising downloading the public health care criteria to the website from the public health agency.

12. The method of providing information to the public health agency as in claim 1 further comprising downloading the public health care criteria to the website from the public health agency based upon a locale of the patient served by the physician.

13. An apparatus for providing information to a public health agency from a website of a physician, such apparatus comprising:
means for detecting entry of a symptom from a patient through the website;
means for comparing the detected symptom with a public health care criteria; and
means for forwarding the detected symptom to the public health agency when the detected symptom meets the public health care criteria.

14. The apparatus for providing information to the public health agency as in claim 13 further comprising means for downloading a webpage to the patient for entry of symptoms through the website.

15. The apparatus for providing information to the public health agency as in claim 14 wherein the means for detecting entry of the symptom from the patient further comprises means for retrieving patient information from predetermined fields of a message from the patient.

16. The apparatus for providing information to the public health agency as in claim 15 further comprising means for performing word searching on the information from the patient.

17. The apparatus for providing information to the public health agency as in claim 16 further comprising means for using words from the healthcare criteria as search terms for the word searching.

18. The apparatus for providing information to the public health agency as in claim 17 wherein the means for forwarding the detected symptom further comprises means for forwarding the information entered by the patient that met the public health care criteria.

19. The apparatus for providing information to the public health agency as in claim 17 further comprising defining the public health care criteria as being a fever above a predetermined value.

20. The apparatus for providing information to the public health agency as in claim 13 further comprising defining the public health care criteria as being a plurality of disease profiles.

21. The apparatus for providing information to the public health agency as in claim 13 further comprising defining the public health care criteria as an adverse event related to the patient's use of a drug or medical device.

22. The apparatus for providing information to the public health agency as in claim 13 further comprising means for reporting the adverse event to a pharmaceutical company sponsoring the drug or to a developer of the medical device.

23. The apparatus for providing information to the public health agency as in claim 15 further comprising means for downloading the public health care criteria to the website from the public health agency.

24. The apparatus for providing information to the public health agency as in claim 15 further comprising means for downloading the public health care criteria to the website from the public health agency based upon a locale of the patient served by the physician.

25. An apparatus for providing information to a public health agency from a website of a physician, such apparatus comprising:
a patient interface adapted to detect entry of a symptom from a patient;
a symptom comparator adapted to compare the detected symptom with a public health care criteria; and
a communication processor adapted to forward the detected symptom to the public health agency when the detected symptom meets the public health care criteria.

26. The apparatus for providing information to the public health agency as in claim 25 further comprising a website adapted to download a webpage to the patient for entry of symptoms.

27. The apparatus for providing information to the public health agency as in claim 25 wherein the public health criteria further comprising descriptive words adapted for use as search terms.

28. The apparatus for providing information to the public health agency as in claim 25 further comprising defining the public health care criteria as being a fever above a predetermined value.

29. The apparatus for providing information to the public health agency as in claim 25 further comprising defining the public health care criteria as being a plurality of disease profiles.
